Europäisches Patentamt

European Patent Office

Office européen des brevets

(19)

(11) EP 1 418 184 A1

(12) **EUROPEAN PATENT APPLICATION**

(43) Date of publication:
**12.05.2004 Bulletin 2004/20**

(51) Int Cl.⁷: **C07K 14/54**, C07K 16/28,
C07K 19/00, C12N 5/00,
A61K 38/17, A61P 35/00

(21) Application number: **02025124.5**

(22) Date of filing: **08.11.2002**

(84) Designated Contracting States:
**AT BE BG CH CY CZ DE DK EE ES FI FR GB GR
IE IT LI LU MC NL PT SE SK TR**
Designated Extension States:
**AL LT LV MK RO SI**

(71) Applicant: **Cell Center Cologne GmbH
50931 Köln (DE)**

(72) Inventors:
• **Hombach, Andreas
50321 Brühl (DE)**

• **Abken, Hinrich
56414 Meudt (DE)**
• **Heuser, Andrea
69469 Weinheim (DE)**

(74) Representative: **MÜLLER FOTTNER STEINECKE
Rechtsanwälte Patentanwälte
Postfach 31 01 40
80102 München (DE)**

(54) **Recombinant fusion protein consisting of the p40 and p35 subunits of IL-12 and a ScFv and use thereof**

(57)     The present invention relates to a recombinant fusion protein as well as to nucleic acids encoding same. The invention is further directed to vectors and host cells containing said nucleic acids as well as to pharmaceutical and diagnostic compositions and their use in the diagnosis and therapy of malignant diseases. The invention further relates to methods of producing the recombinant fusion protein.

Figure 1

HRS3-scFv                    single-chain IL-12

| VH | L | VL | EPKSPDKTHTCPP | p40 | L | p35 |

human IgG hinge

SEQUENCE LISTING

<110>  Cell Center Cologne GmbH

<120>  Recombinant fusion protein and use thereof

<130>  P14706

<160>  4

<170>  PatentIn version 3.1

<210>  1
<211>  39
<212>  DNA
<213>  Homo sapiens

<400>  1
gagcccaaat ctcctgacaa aactcataca tgcccacca
      39


<210>  2
<211>  76
<212>  DNA
<213>  Artificial sequence

<220>
<223>  Oligonucleotide primer

<400>  2
gaaggatccc gccgagccca atctcctga caaaactcat acatgcccac caatgtggga
      60

gctggagaaa gacgtt
      76


<210>  3
<211>  33
<212>  DNA
<213>  Artificial sequence

<220>
<223>  Oligonucleotide primer

<400>  3
acttctctcg agtcaggcgg agctcagata gcc
      33


<210>  4
<211>  2438

```
<212>    DNA
<213>    Artificial sequence

<220>
<223>    recombinant fusion protein coding sequence

<400>   4
tacgtaccat ggattttcag gtgcagattt tcagcttcct gctaatcagt gcctcagtca
      60

taatgtctag aatggcccag gtgcaactgc agcagtcagg ggctgagctg ctagacctg
      120

gggcttcagt gaagatgtcc tgcaaggctt ctggctacac ctttactacc tacacaatac
      180

actgggtaag acagaggcct ggacacgatc tggaatggat tggatacatt aatcctagca
      240

gtggatattc tgactacaat cagaacttca agggcaagac cacattgact gcagacaagt
      300

cctccaacac agcctacatg caactgaaca gcctgacatc tgaggactct gcggtctatt
      360

actgtgcaag aagagcggac tatggtaact acgaatatac ctggtttgct tactggggcc
      420

aagggaccac ggtcaccgtc tcctcaggtg gaggcggttc aggcggaggt ggctctggcg
      480

gtggcggatc ggacatcgag ctcactcagt ctccaaaatt catgtccaca tcagtaggag
      540

acagggtcaa cgtcacctac aaggccagtc agaatgtggg tactaatgta gcctggtttc
      600

aacaaaaacc agggcaatct cctaaagttc tgatttactc ggcatcttac cgatacagtg
      660

gagtccctga tcgcttcaca ggcagtggat ctggaacaga tttcactctc accatcagca
      720

atgtgcagtc tgaagacttg gcagagtatt tctgtcagca atatcacacc tatcctctca
      780

cgttcggagg gggcaccaag ctggaaatca aacgggcgga tcccgccgag cccaaatctc
      840

ctgacaaaac tcatacatgc ccaccaatgt gggagctgga gaaagacgtt tatgttgtag
      900

aggtggactg gactcccgat gcccctggag aaacagtgaa cctcacctgt gacacgcctg
```

960

aagaagatga catcacctgg acctcagacc agagacatgg agtcataggc tctggaaaga
1020

ccctgaccat cactgtcaaa gagtttctag atgctggcca gtacacctgc cacaaaggag
1080

gcgagactct gagccactca catctgctgc tccacaagaa ggaaaatgga atttggtcca
1140

ctgaaatttt aaaaaatttc aaaaacaaga ctttcctgaa gtgtgaagca ccaaattact
1200

ccggacggtt cacgtgctca tggctggtgc aaagaaacat ggacttgaag ttcaacatca
1260

agagcagtag cagtccccccc gactctcggg cagtgacatg tggaatggcg tctctgtctg
1320

cagagaaggt cacactggac caaagggact atgagaagta ttcagtgtcc tgccaggagg
1380

atgtcacctg cccaactgcc gaggagaccc tgcccattga actggcgttg gaagcacggc
1440

agcagaataa atatgagaac tacagcacca gcttcttcat caggacatc atcaaaccag
1500

acccgcccaa gaacttgcag atgaagcctt tgaagaactc acaggtggag gtcagctggg
1560

agtaccctga ctcctggagc actccccatt cctacttctc cctcaagttc tttgttcgaa
1620

tccagcgcaa gaaagaaaag atgaaggaga cagaggaggg gtgtaaccag aaaggtgcgt
1680

tcctcgtaga gaagacatct accgaagtcc aatgcaaagg cgggaatgtc tgcgtgcaag
1740

ctcaggatcg ctattacaat tcctcatgca gcaagtgggc atgtgttccc tgcagggtcc
1800

gatccggtgg cggtggctcg ggcggtggtg ggtcgggtgg cggcggatct agggtcattc
1860

cagtctctgg acctgccagg tgtcttagcc agtcccgaaa cctgctgaag accacagatg
1920

acatggtgaa gacggccaga gaaaaactga acattattc ctgcactgct gaagacatcg
1980

```
atcatgaaga catcacacgg gaccaaacca gcacattgaa gacctgttta ccactggaac
   2040

tacacaagaa cgagagttgc ctggctacta gagagacttc ttccacaaca agagggagct
   2100

gcctgccccc acagaagacg tctttgatga tgaccctgtg ccttggtagc atctatgagg
   2160

acttgaagat gtaccagaca gagttccagg ccatcaacgc agcacttcag aatcacaacc
   2220

atcagcagat cattctagac aagggcatgc tggtggccat cgatgagctg atgcagtctc
   2280

tgaatcataa tggcgagact ctgcgccaga aacctcctgt gggagaagca gacccttaca
   2340

gagtgaaaat gaagctctgc atcctgcttc acgccttcag cacccgcgtc gtgaccatca
   2400

acagggtgat gggctatctg agctccgcct gactcgag
   2438
```

**Description**

The present invention relates to a recombinant fusion protein as well as to nucleic acids encoding same. The invention is further directed to vectors and host cells containing said nucleic acids as well as to pharmaceutical and diagnostic compositions and their use in the diagnosis and therapy of malignant diseases. The invention further relates to methods of producing the recombinant fusion protein.

Interleukin-12 (IL-12) is a disulfide-linked heterodimeric cytokine composed of a 35 kDa light chain (p35) and a 40 kDa heavy chain (p40) and is predominantly produced by activated macrophages and dendritic cells. IL-12 is an extremly pleiotropic cytokine and plays a key role in linking innate cellular immunity to an adoptive Th1 response against pathogens and tumor cells [1] by counteracting a Th2 immune response [2]. IL-12 production by antigen presenting cells and its function on immune cells, however, is negatively regulated by the Th2 cytokines IL-10 and TGF-β [3, 4]. These cytokines are, for example, also present in lesions with Hodgkin's disease [5] suggesting that they are involved in the immunosuppression frequently observed in patients with Hodgkin's disease and other malignancies [6-8].

Hodgkin's disease is characterized by a small number of malignant cells, comprising less than 1% of the tumor mass, that are accompanied by a massive infiltration with benign cells, i. e., T cells, B cells, monocytes, eosinophils and others [9]. In the majority of cases the malignant Hodgkin- and Reed-Sternberg (H/RS) tumor cell is derived from germinal center B cells [10] and secretes a variety of cyto- and chemokines that are thought to be involved in the pathogenesis and in clinical symptoms of the disease [11]. Most of these cytokines are known to favor a Th2 immune response that in turn may favor an impaired cellular anti-tumor reactivity [5, 12-17]. Despite a prominent local immune reaction in the malignant tissues, the general immune response in patients with Hodgkin's lymphoma paradoxically exhibits symptomes of an acquired cellular immune deficiency [17] and a Th2 imbalance.

Accordingly, elevated levels of serum IL-10 are associated with a poor prognosis of HD patients [18].

The heterodimeric structure of IL-12 (p35, p40) makes the generation of functionally active antibody-IL-12 fusion proteins more difficult. During the last years, several strategies were used: (i) The p35 IL-12 subunit was fused to the C-terminus of the human IgG1 or IgG3 heavy chain and coexpressed with the heterologous p40 IL-12 subunit and the homologous IgG light chain, respectively. The resulting recombinant immunoglobulins partly assembled to IgG-p35/p40 heterodimers but retained substantial lower IL-12 bioreactivity than the natural cytokine [19]. (ii) The p35 IL-12 subunit was directly fused to the C-terminus of a scFv and coexpressed with the p40 subunit. The resulting scFv-p35/p40 protein had impaired IL-12 reactivity similarly as the IgG-p35/p40 fusion protein [19]. (iii) Since the accessability of the p40 N-terminus was postulated to be required for functional IL-12 activity, recombinant (p40-p35) single chain IL-12 was fused to the N-terminus of an IgG3 heavy chain. Coexpression of this fusion protein with its corresponding IgG light chain resulted in a recombinant antibody with both specific antigen binding and high IL-12 reactivity [20]. Taken together, the above summarized approaches require coexpression and functional assembly of at least two different polypeptide chains to create a functionally active antibody-IL-12 cytokine fusion protein.

To avoid coexpression of two different polypeptide chains and to facilitate the expression of recombinant IL-12 fusion proteins, the use of single chain IL-12 consisting either of p40-p35 or p35-p40 was suggested. P35-p40 single chain IL-12, whose p40 domain was fused via the N-terminus and a flexible linker to the carboxy-terminus of the p35 subunit, was demonstrated to be not functional [21]. Engraftment of p35-p40 single chain IL-12 with an additional IL-2 effector domain resulted in proliferative capacity on activated T cells that can be attributed to the IL-2 domain [22] and application of this IL-12 fusion protein may interfere with stimulation of the IL-2 receptor. To provide free accessibility of the p40 IL-12 domain that is attributed with high IL-12 activity, a recombinant p40-p35 single chain IL-12 was fused to the N-terminus of an antibody derived scFv [23]. Although the resulting fusion protein retained IL-12 activity, this configuration is limited for ligands that do not need a free N-terminus of the scFv. The range of possible binding domains thus is restricted in this approach to such ligands, which do not require a free N-terminus for functional expression. The production of single chain fusion proteins having IL-12 action without additional cytokine-derived effector domains, in which a coupling domain for specific ligand binding is covalently bound to the N-terminus of the p40-subunit has not been described before.

Therefore, it is an object of the present invention to provide a novel class of IL12 fusion proteins for the immunostimulatory therapy, which avoid the drawbacks of coexpression and functional assembly of different polypeptides and which retain full activity of their components.

This object is solved by the subject-matters of the independent claims. Preferred embodiments are set forth in the dependent claims.

The solution according to the present invention resists in the production of a single polypeptide chain having IL-12 action, in which a polypeptide, for example a single chain antibody or other molecule is covalently bound to the N-terminus of the p40-subunit of the IL-12, which may in turn be covalently bound to a p40- or p35 IL-12 subunit. The covalent coupling of that domain to the N-terminus of the p40 subunit can be achieved directly or using a linker peptide. Thus, recombinant proteins consisting of a single polypeptide chain and including a fusion of a polypeptide or other molecule to the N-terminus of the p40-subunit of IL-12 and achieving a functional binding to the IL-12 receptor can be manufactured.

The approach of the present invention shows the following surprising results, which distinguish the fusion proteins of the present invention from those of the prior art approaches. The recombinant fusion proteins of the invention maintain the biological activity of each component, i.e. the coupling domain for specific ligand binding as well as the single chain IL-12, which property is attributable to the coupling of the inventive components via the N-terminus of the p40 subunit of IL-12. The prior art constructs that were coupled via the N-terminus to the p40 subunit were said not to be immunologically effective [21].

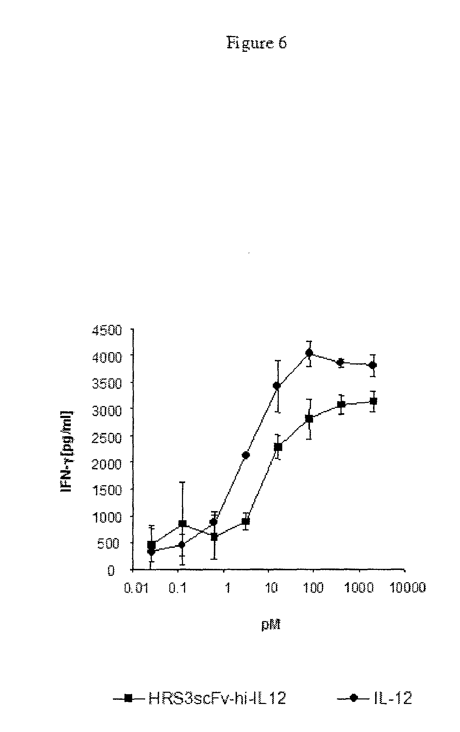

As an example, to facilitate the generation and expression of modularily composed antibody-IL-12 cytokine fusion proteins, the inventors fused the scFv domain via a human IgG1-hinge derived linker to the N-terminus of the p40 subunit of IL-12. The resulting fusion protein combines both properties, IL-12 activity and specific binding via the scFv antibody. In terms of induction of IFN-$_\gamma$ secretion in T cells, the IL-12 activity is only slightly lower than that of the natural cytokine (cf. Fig. 6). The data moreover indicate that the free accessibility of the p40 IL-12 subunit is not required for proper function of the fusion protein but, however, may facilitate binding to and subsequent signalling through the IL-12 receptor.

The findings presented herein have substantial consequences for the generation of recombinant antibody-IL-12 cytokine fusion proteins: (i) once generated, the domains of modularily composed recombinant single chain IL-12 fusion proteins can be easily substituted and rearranged to form proteins with desired binding specificity and bioactivity. (ii) A slightly lower bioactivity of the IL-12 domain may detoxify the molecule in part making it more suitable for systemic application. High accumulation of IL-12 via the antigen-binding domain at the tumor site, however, will compensate for a lower bioactivity and provide site specific stimulation of effector cells.

According to a first aspect, the present invention provides a recombinant fusion protein, which comprises the following functionally linked components:

a) a coupling domain for specific ligand binding,
b) a single chain IL-12 or a fragment thereof, wherein the fragment at least contains the p40 subunit of IL-12, and
c) optionally a peptide linker, which links components (a) and (b) to each other,

wherein the single chain IL-12 or the fragment thereof is linked to the other components via the N-terminus of the p40 subunit.

One essential component of the fusion protein of the invention is a coupling domain for specific ligand binding. That domain may preferably comprise a coupling domain which is selected from a group consisting of polypeptides, antibodies, antibody fragments, therapeutic agents, dyes, toxins or chelating agents. A "specific ligand binding" is defined herein as binding of molecule A to molecule B resulting in a complex consisting of A and B.

As mentioned above, the coupling domain may, for example, consist of an antibody. The term "antibody" as used herein refers to intact antibodies as well as antibody fragments that retain some ability to selectively bind an epitope. Such fragments include, without limitation, Fab, F(ab')$_2$, and Fv antibody fragments. The term "epitope" refers to an antigenic determinant on an antigen to which the idiotope of an antibody binds. Epitopic determinants usually consist of chemically active surface groupings of molecules (e. g., amino acid or sugar residues) and usually have specific three dimensional structural characteristics as well as specific charge characteristics.

The antibody may be a "humanized" monoclonal antibody. Humanized monoclonal antibodies are produced by transferring mouse complementarity determining regions (CDRs) from heavy and light variable chains of the mouse immunoglobulin into a human variable domain, and then substituting human residues in the framework regions of the murine counterparts. The use of antibody components derived from humanized monoclonal antibodies obviates potential problems associated with the immunogenicity of murine constant regions when treating humans. General techniques for cloning murine immunoglobulin variable domains are described, for example, by Orlandi et al., Proc. Nat'l. Acad. Sci. USA 86: 3833 (1989). Techniques for producing humanized monoclonal antibodies are described, for example, by Jones et al., Nature 321: 522 (1986); Riechmann et al., Nature 332: 323 (1988); Verhoeyen et al., Science 239: 1534 (1988); Carter et al., Proc. Nat'l. Acad. Sci. USA 89: 4285 (1992); Sandhu, Crit. Rev. Biotech. 12: 437 (1992); and Singer et al., J : Immunol. 150: 2844 (1993).

Antibodies of the present invention also may be derived from human antibody fragments isolated from a combinatorial immunoglobulin library. See, for example, Barbas et al., Methods: A Companion To Methods In Enzymology, Vol. 2, page 119 (1991) and Winter et al., Ann. Rev. Immunol. 12: 433 (1994). Cloning and expression vectors that are useful for producing a human immunoglobulin phage library can be obtained, for example, from STRATAGENE Cloning Systems (La Jolla, CA, USA).

In addition, antibodies of the present invention may be derived from a human monoclonal antibody. Such antibodies are obtained from transgenic mice that have been "engineered" to produce specific human antibodies in response to antigenic challenge. In this technique, elements of the human heavy and light chain loci are introduced into strains of mice derived from embryonic stem cell lines that contain targeted disruptions of the endogenous heavy and light chain loci. The transgenic mice can synthesize human antibodies specific for human antigens and can be used to produce human antibody-secreting hybridomas. Methods for obtaining human antibodies from transgenic mice are described by Green et al., Nature Genet. 7: 13 (1994); Lonberg et al., Nature 368: 856 (1994); and Taylor et al., Int. Immunol. 6: 579 (1994).

All mentioned antibodies may additionally be linked to a toxic agent and/or to a detectable agent.

According to a preferred embodiment, the antibody fragment is a scFv. A scFv antibody fragment is an engineered antibody derivative that includes heavy- and light-chain variable regions joined by a peptide linker. ScFv antibody fragments are potentially more effective than unmodified antibodies. The reduced size of approx. 27—30 kDa permits them to penetrate tissues and solid tumors more readily.

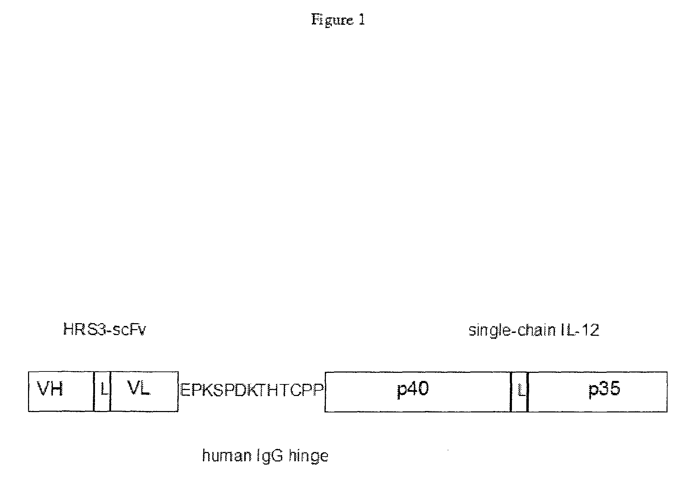

According to a further preferred embodiment, the scFv is an anti-CD 30 scFv. The CD30 antigen is commonly expressed on activated T, B, NK cells, monocytes and tumor cells like Hodgkin Lymphoma cells. Its molecular weight is 120 kDa. Cross-linking CD30 enhances proliferation of Band T cells. Most preferably, the anti-CD 30 scFv is the HRS3-scFv directed against Hodgkin-Reed-Sternberg (HRS) cells. That CD30 antigen binding domain is a single chain fragment of variable regions (scFv) derived from the anti-CD30 mAb HRS3. Thus, an in particular preferred fusion protein of the present invention has the configuration shown in Fig. 1.

As mentioned above, the components a) and b) of the fusion protein of the invention can be directly, i.e. covalently coupled or can be connected via a peptide linker. This linker preferably is the human IgG1 hinge region. The precise sequence of the hinge region is GAGCCCAAATCTCCTGACAAAACTCATACATGCCCACCA (SEQ ID NO: 1).

According to a further aspect, the fusion protein of the invention is encoded by the nucleic acid of SEQ ID NO: 4.

The present invention further provides an isolated nucleic acid, which is coding for the fusion protein of the present invention.

According to an embodiment of the invention, the nucleic acids of the present invention comprise transcriptional products of one of the above nucleic acids, e.g. mRNA, as well as nucleic acids, which selectively hybridize to said transcriptional products of the nucleic acids under moderate stringent conditions (definition see below).

The term "nucleic acid sequence" refers to a heteropolymer of nucleotides or the sequence of these nucleotides. The terms "nucleic acid" and "polynucleotide" are used interchangeably herein to refer to a heteropolymer of nucleotides.

The polynucleotides of the present invention also include, but are not limited to, a polynucleotide that hybridizes to the complement of the disclosed nucleotide sequences under moderately stringent or stringent hybridization conditions; a polynucleotide which is an allelic variant of any polynucleotide recited above; a polynucleotide which encodes a species homologue of any of the herein disclosed proteins; or a polynucleotide that encodes a polypeptide comprising an additional specific domain or truncation of the disclosed proteins.

Stringency of hybridization, as used herein, refers to conditions under which polynucleotide duplexes are stable. As known to those of skill in the art, the stability of duplex is a function of sodium ion concentration and temperature (see, for example, Sambrook et al., Molecular Cloning: A Laboratory Manual 2$^{nd}$ Ed. (Cold Spring Harbor Laboratory, (1989)). Stringency levels used to hybridize can be readily varied by those of skill in the art.

The phrase "low stringency hybridization" refers to conditions equivalent to hybridization in 10% formamide, 5 x Denhart's solution, 6 x SSPE, 0.2% SDS at 42°C, followed by washing in 1 x SSPE, 0.2% SDS, at 50°C Denhart's solution and SSPE (see, e.g., Sambrook et al., Molecular Cloning, A Laboratory Manual, Cold Spring Harbor Laboratory Press, 1989) are well known to those of skill in the art as are other suitable hybridization buffers.

As used herein, the phrase "moderately stringent hybridization" refers to conditions that permit DNA to bind a complementary nucleic acid that has about 60% identity, preferably about 75% identity, more preferably about 85% identity to the DNA; with greater than about 90% identity to said DNA being especially preferred. Preferably, moderately stringent conditions are conditions equivalent to hybridization in 50% formamide, 5 x Denhart's solution, 5 x SSPE, 0.2% SDS at 42°C, followed by washing in 0.2 x SSPE, 0.2% SDS, at 65°C.

The phrase "high stringency hybridization" refers to conditions that permit hybridization of only those nucleic acid sequences that form stable duplex in 0.018M NaCl at 65°C. (i.e., if a duplex is not stable in 0.018M NaCl at 65.degree°C, it will not be stable under high stringency conditions, as contemplated herein). High stringency conditions can be provided, for example, by hybridization in 50% formamide, 5 x Denhart's solution, 5 x SSPE, 0.2% SDS at 42°C, followed by washing in 0.1 x SSPE, and 0.1% SDS at 65°C.

Any cell containing an isolated nucleic acid within the scope of the invention is itself within the scope of the invention. This includes, without limitation, prokaryotic and eukaryotic cells. It is noted that cells containing an isolated nucleic acid of the invention are not required to express the isolated nucleic acid. In addition, the isolated nucleic acid can be integrated into the genome of the cell or maintained in an episomal state. In other words, cells can be stably or transiently transfected with an isolated nucleic acid of the invention.

Generally, the term "nucleic acid" as used herein encompasses both RNA and DNA, including cDNA, genomic DNA, and synthetic (e. g., chemically synthesized) DNA.

The nucleic acid can be double-stranded or single-stranded. Where single-stranded, the nucleic acid can be the sense strand or the antisense strand. In addition, nucleic acid can be circular or linear.

The term "isolated" as used herein with reference to nucleic acid refers to a naturally-occurring nucleic acid that is not immediately contiguous with both of the sequences with which it is immediately contiguous (one on the 5'end and one on the 3'end) in the naturally-occurring genome of the organism from which it is derived.

For example, an isolated nucleic acid can be, without limitation, a recombinant DNA molecule of any length, provided one of the nucleic acid sequences normally found immediately flanking that recombinant DNA molecule in a naturally-occurring genome is removed or absent. Thus, an isolated nucleic acid includes, without limitation, a recombinant DNA that exists as a separate molecule (e. g., a cDNA or a genomic DNA fragment produced by PCR or restriction endonuclease treatment) independent of other sequences as well as recombinant DNA that is incorporated into a vector, an autonomously replicating plasmid, a virus (e. g., a retrovirus, adenovirus, or herpes virus), or into the genomic DNA of a prokaryote or eukaryote. In addition, an isolated nucleic acid can include a recombinant DNA molecule that is part of a hybrid or fusion nucleic acid sequence.

The term "isolated" also includes any non-naturally-occurring nucleic acid since non-naturally-occurring nucleic acid sequences are not found in nature and do not have immediately contiguous sequences in a naturally-occurring genome. For example, non-naturally-occurring nucleic acid such as an engineered nucleic acid is considered to be isolated nucleic acid. Engineered nucleic acid can be made using common molecular cloning or chemical nucleic acid synthesis techniques. Isolated non-naturally-occurring nucleic acids can be independent of other sequences, or incorporated into a vector, an autonomously replicating plasmid, a virus (e. g., a retrovirus, adenovirus, or herpes virus), or the genomic DNA of a prokaryote or eukaryote. In addition, a non-naturally-occurring nucleic acid can include a nucleic acid molecule that is part of a hybrid or fusion nucleic acid sequence.

It will be apparent to those of skill in the art that a nucleic acid existing among hundreds to millions of other nucleic acid molecules within, for example, cDNA or genomic libraries, or gel slices containing a genomic DNA restriction digest is not to be considered an isolated nucleic acid.

According to a further aspect, the present invention provides an isolated nucleic acid, which selectively hybridizes to the aforementioned nucleic acids under moderately stringent conditions. For a definition of the term "moderately stringent conditions", see above.

According to a further aspect of the invention, a vector is provided which comprises the above mentioned nucleic acids. Preferably, an expression vector is provided, which comprises said nucleic acids coding for a recombinant fusion protein of the invention. This expression vector preferably comprises one or more regulatory sequences. The term "expression vector" generally refers to a plasmid, cosmid, bacteriophage or a virus or other vector, for expressing a polypeptide from a DNA (RNA) sequence. An expression vector can comprise a transcriptional unit comprising an assembly of (i) a genetic element or elements having a regulatory role in gene expression, for example, promoters or enhancers, (ii) a structural or coding sequence which is transcribed into mRNA and translated into protein, and (iii) appropriate transcription initiation and termination sequences. Structural units intended for use in yeast or eukaryotic expression systems preferably include a leader sequence enabling extracellular secretion of translated protein by a host cell. Alternatively, where recombinant protein is expressed without a leader or transport sequence, it may include an N-terminal methionine residue. This residue may or may not be subsequently cleaved from the expressed recombinant protein to provide a final product.

The present invention further provides hosts, e.g. host cells, which have been transformed to contain the polynucleotides of the invention. The term "transformation" means introducing DNA into a suitable host cell so that the DNA is replicable, either as an extrachromosomal element, or by chromosomal integration.

For example, such host cells may contain nucleic acids of the invention introduced into the host cell using known transformation methods. The present invention still further provides host cells genetically engineered to express the polynucleotides of the invention, wherein such polynucleotides are in operative association with a regulatory sequence heterologous to the host cell which drives expression of the polynucleotides in the cell.

The host cell can be a higher eukaryotic host cell, such as a mammalian cell, a lower eukaryotic host cell, such as a yeast cell, or can be an insect cell, or the host cell can be a prokaryotic cell, such as a bacterial cell. Introduction of the recombinant construct into the host cell can be effected by calcium phosphate transfection, DEAE, dextran mediated transfection, or electroporation (Davis, L. et al., Basic Methods in Molecular Biology (1986)).

The most preferred cells are those which do not normally express the particular polypeptide or protein or which expresses the polypeptide or protein at low natural level. Mature proteins can be expressed in mammalian cells, yeast, bacteria, plant, insect or other cells under the control of appropriate promoters. Appropriate cloning and expression vectors for use with prokaryotic and eukaryotic hosts are described by Sambrook, et al., in Molecular Cloning: A Laboratory Manual, 2nd Edition, Cold Spring Harbor, New York (1989).

The mammalian cell is pereferably a CHO, COS, HeLa, 293T, HEH or BHK cell. As procaryotic cells, *E.coli* and *Bacillus subtilis* are preferred.

According to a further aspect, the present invention provides a pharmaceutical composition comprising a therapeutically effective amount of the fusion protein, the nucleic acids or of the vectors of the present invention, and a pharmaceutically acceptable carrier.

The ingredients of the present invention are preferably used in form of a pharmaceutical composition where they are mixed with suitable carriers or excipients in doses to treat or ameliorate the disease. Such a composition may also contain (in addition to the ingredient and the carrier) diluents, fillers, salts, buffers, stabilizers, solubilizers and other materials well known in the art. The term "pharmaceutically acceptable" means a non-toxic material that does not interfere with the effectiveness of the biological activity of the active ingredient(s). The characteristics of the carrier will depend on the route of administration. The pharmaceutical composition may further contain other agents which either enhance the activity or use in treatment. Such additional factors and/or agents may be included in the pharmaceutical composition to produce a synergistic effect or to minimize side-effects.

Techniques for formulation and administration of the compounds of the instant application may be found in "Remington's Pharmaceutical Sciences", Mack Publishing Co., Easton, PA, latest edition. Whenever the compositions are to be used for medical purposes, they will contain a therapeutically effective dose of the respective ingredient. A therapeutically effective dose further refers to that amount of the compound/ingredient sufficient to result in amelioration of symptoms, e.g., treatment, healing, prevention or amelioration of such conditions. Suitable routes of administration may, for example, include oral, rectal, transmucosal, or intestinal administration; parenteral delivery, including intramuscular, subcutaneous, intramedullary injections, as well as intrathecal, direct intraventricular, intravenous, intraperitoneal or intranasal injections. Administration of the fusion protein/nucleic acids comprised in the pharmaceutical composition of the present invention can be carried out in a variety of conventional ways, such as oral ingestion, inhalation, topical application or cutaneous, subcutaneous, intraperitoneal, parenteral or intravenous injection. Intravenous administration to the patient is preferred.

A typical composition for intravenous infusion can be made up to contain 250 ml of sterile Ringer's solution, and 1-10 mg of the fusion protein of the present invention. See Remington's Pharmaceutical Science (for example 15th Ed., Mack Publishing Company, Easton, Ps., 1980).

According to a preferred embodiment, in the pharmaceutical composition of the invention the fusion protein is further coupled to a therapeutic agent. Preferably, the fusion protein is coupled to substances improving the serum half life of the fusion protein, i. e. albumine.

Furthermore, the fusion protein may be coupled to a radioisotope, a "biological response modifier", e. g. IL-2, IL-4, TNF-alpha, enzymes, e. g. for prodrug activation, coupling domains for engraftment of radioisotypes and drugs, e. g. cytostatic drugs.

According to a preferred embodiment, the pharmaceutical composition is a vaccine.

The above mentioned pharmaceutical composition finds application in the immunotherapy of malignant diseases, preferably of the Hodgkin lymphoma, minimal residual disease, inflammatory diseases, allergic and rheumatoid diseases, tumor prevention, of preneoplastic lesions and suppression of a Th2-immune response mediated by CD30+ immunological competent cells.

According to a further aspect, the present invention provides a diagnostic composition comprising the fusion protein of the invention, wherein the fusion protein is detectably labelled. Detectable labellings in accordance with the present invention are, for example, radioactive labelling, fluorescent labelling, biotin, digoxigenin or peroxidase labellings or a labelling, which is detectable by means of alkaline phosphatase. Preferably, the fusion protein is radio labelled, more preferably labelled with radioactive iodine.

The diagnostic composition of the present invention can be used in the diagnosis of CD30+ diseases, preferably of the Hodgkin lymphoma, minimal residual disease, inflammatory diseases, allergic and rheumatoid diseases, preneoplastic lesions and of a Th2-immune response mediated by CD30+ immunological competent cells.

According to a further aspect of the present invention, a method of producing a substantially pure fusion protein of the present invention is provided, comprising transforming a host cell of the invention with a vector as mentioned above, cultivating said host cell under conditions, which allow an expression of the vector by the host cell, and isolating the fusion protein from the host cell. The methods for producing a substantially pure fusion protein comprises growing a culture of the cells of the invention in a suitable culture medium, and purifying the protein from the culture. For example, the methods of the invention include a process for producing a protein in which a host cell containing a suitable expression vector that encodes a protein of the invention is cultured under conditions that allow expression of the encoded fusion protein. The fusion protein can be recovered from the culture, conveniently from the culture medium, and can be further purified by affinity chromatography utilizing anti-idiotypic antibodies. The resulting expressed protein may for example be purified from such culture (i.e., from culture medium or cell extracts) using known purification processes, such as gel filtration and ion exchange chromatography.

Alternatively, a method of producing a substantially pure fusion protein according to the invention comprises the steps of:

a) providing a coupling domain for specific ligand binding and a single chain IL-12 or a fragment thereof, wherein the fragment at least contains the p40 subunit of IL-12,

b) coupling the coupling domain via a peptide bond to the N-terminus of the p40 subunit of the single chain IL-12 or the fragment thereof, and

(c) isolating the obtained fusion protein.

Unless otherwise defined, all technical and scientific terms used herein have the same meaning as commonly understood by one of ordinary skill in the art to which this invention pertains. All publications and other references mentioned herein are incorporated by reference in their entirety. In case of conflict, the present specification, including definitions, will control. In addition, the materials, methods, and examples are illustrative only and not intended to be limiting.

The invention is now further illustrated by the accompanying drawings and examples. The drawings are showing the following:

Fig. 1 is a schematic diagram of the HRS3-scFv-hi-IL-12 single chain fusion protein. The HRS3-scFv is fused via a modified human IgG1-hinge region to the N-terminus of the murine single chain IL-12 p40/p35 fusion protein. VH: variable heavy chain domain; VL: variable light chain domain; L: $(Gly_4Ser)_3$ linker; p35: p35 subunit of IL-12; p40: p40 subunit of IL-12.

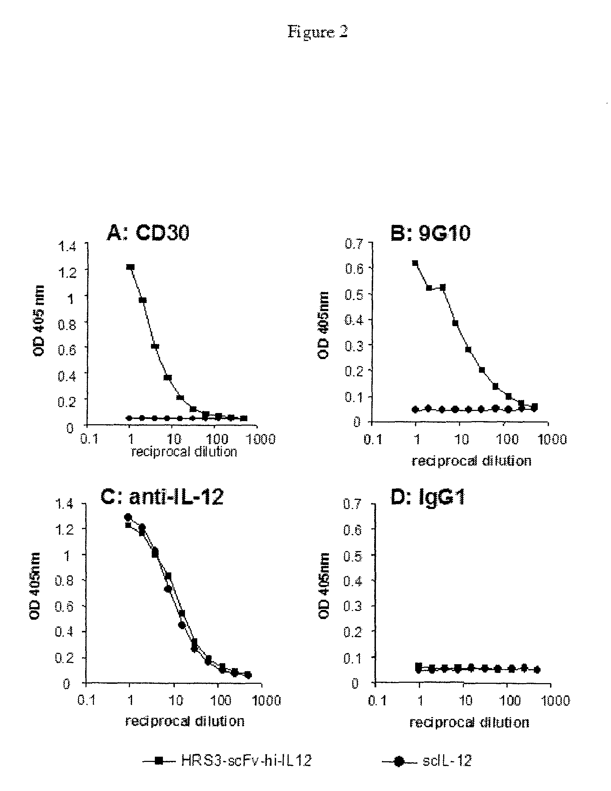

Fig. 2: Characterization of the HRS3-scFv-hi-IL-12 fusion protein. Culture supemantants of 293T cells that were transfected with expression plasmids encoding either the HRS3-scFv-hi-IL-12 or the single chain IL-12 (p40-p35) fusion protein were incubated in microtiter plates coated with the CD30-antigen (A), the HRS3-scFv specific anti-idiotypic mAb 9G10 (B), an anti-mouse IL-12 antibody (C), or an mouse IgG control mAb (D). Bound proteins were detected by a biotinylated anti-mouse IL-12 antibody and streptavidin-conjugated POD.

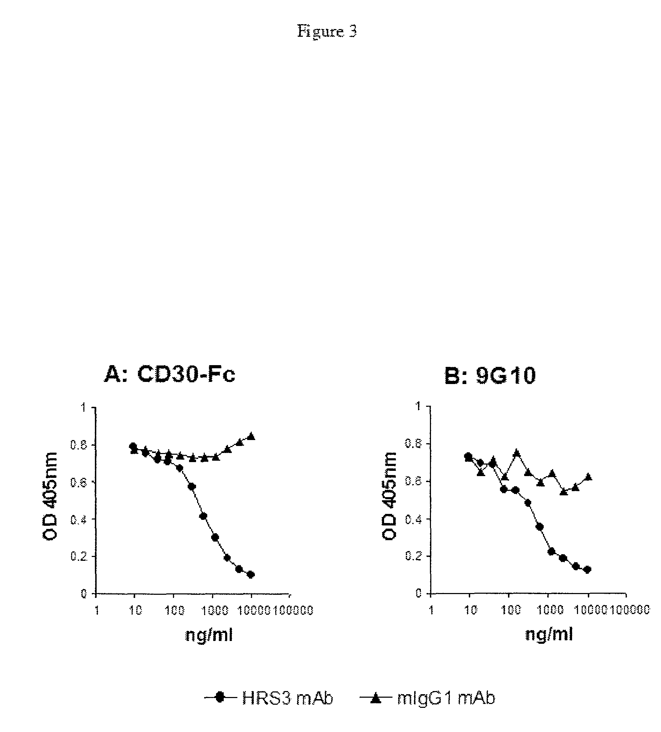

Fig. 3: Specific binding of the HRS3-scFv-hi-IL-12 fusion protein via the scFv domain is blocked by the HRS3 mAb. Culture supernatant containing the HRS3-scFv-hi-IL-12 fusion protein was incubated in microtiter plates that were coated with the CD30 antigen (A) or the HRS3-scFv-specific anti-idiotypic mAb 9G10 (B) in the presence of increasing amounts of the parental mAb HRS3 or an isotype matched control IgG1. Bound proteins were detected by a biotinylated anti-mouse IL-12 antibody and streptavidin-conjugated POD.

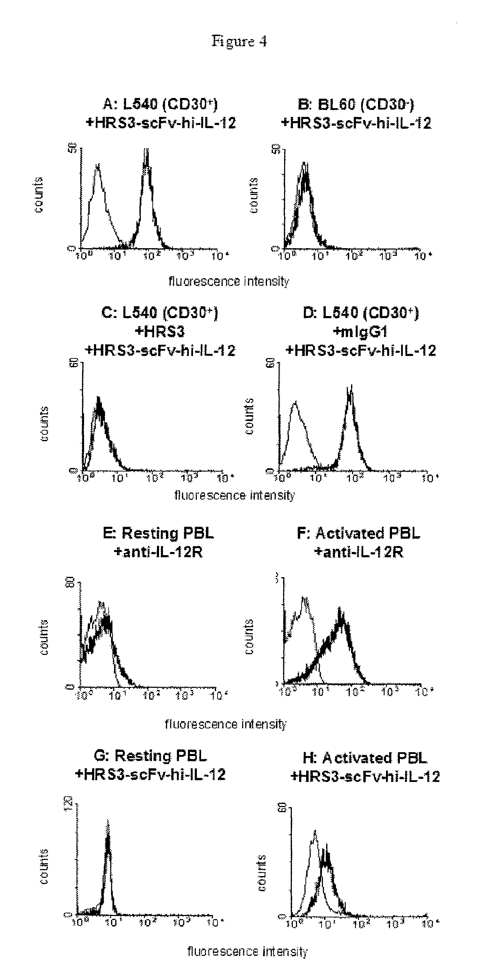

Fig. 4: HRS3-scFv-hi-IL-12 fusion protein binds specifically to CD30$^+$ tumor cells and to the IL-12 receptor on activated lymphocytes. Culture supernatants containing HRS3-scFv-hi-IL-12 (solid lines) or medium (thin lines) were incubated with CD30$^+$ L540 (A) and CD30$^-$ BL60 (B) tumor cells, respectively, or incubated with CD30$^+$ L540 cells in the presence of 20 μg/ml of the anti-CD30 mAb HRS3 (C) or an isotype control mAb (D). Cell bound fusion protein was detected by a biotin-conjugated anti-mouse IL-12 mAb and FITC-conjugated streptavidin by flow cytometry.

Quiescent (E) or preactivated peripheral blood lymphocytes (PBL) (F) were incubated with a PE-conjugated anti-IL-12Rβ1 (solid line) or a PE-conjugated IgG1 control (thin line) antibody to monitor IL-12 receptor expression. Quiescent (G) or preactivated PBL (H) were incubated with culture supernatants containing the HRS3-scFv-hi-IL-12 fusion protein. Bound fusion protein was detected by incubation with the anti-HRS3 scFv idiotypic mAb 9G10 (solid lines) or an IgG1 control antibody (thin lines) and a FITC-conjugated anti-mouse IgG. Cells were analysed by flow cytometry and histograms were overlayed.

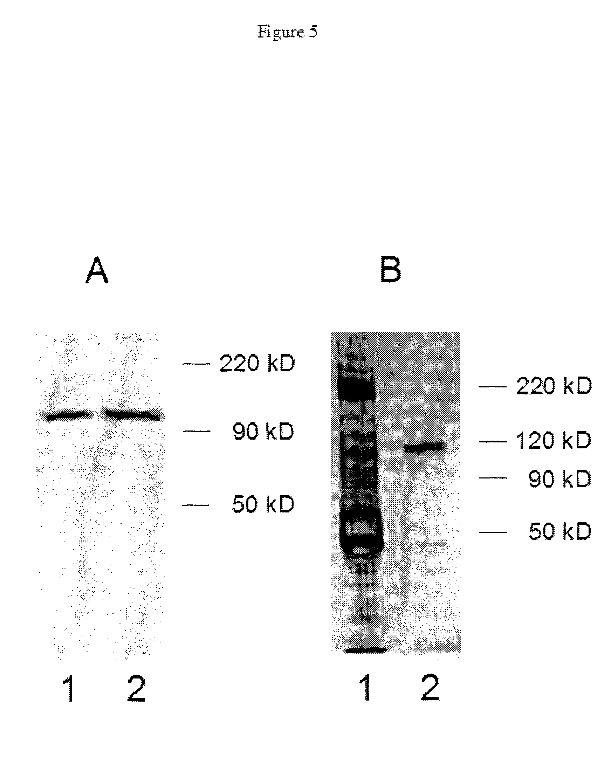

Fig. 5: SDS-PAGE and Western-Blot analysis of the recombinant HRS3-scFv-hi-IL-12 fusion protein.

(A) Culture supernatant containing HRS3-scFv-hi-IL-12 was seperated electrophoretically in a 8% polyacrylamide gel under reducing (lane 1) and non-reducing conditions (lane 2) and blotted onto nitrocellulose filters. The HRS3-scFv-hi-IL-12 fusion protein was detected with a biotin-conjugated anti-mouse IL-12 antibody as described in Materials and Methods.

(B) Culture supernatant containing the HRS3-scFv-hi-IL-12 fusion protein (lane 1) and the affinity purified fusion protein (lane 2), respectively, were seperated in a 4-15% gradient polyacrylamide gel and protein bands were visualized by silver staining as described in Materials and Methods.

Fig. 6: The purified HRS3-scFv-hi-IL-12 fusion protein induces IFN-γ secretion. Preactivated peripheral blood lymphocytes were incubated in microtiter plates ($1 \times 10^5$ cells/well) in the presence of equimolar amounts of the purified HRS3-scFv-hi-IL-12 fusion protein or of natural IL-12. After incubation for 48 h, the culture supernatants were harvested and IFN-γ secretion was detected by ELISA.

Fig. 7: HRS3-scFv-hi-IL-12 fusion protein bound to CD30$^+$ tumor cells induces IFN-γ secretion in peripheral blood lymphocytes.

(A) CD30$^+$ L540 and CD30$^-$ BL60 cells ($1 \times 10^6$) were incubated in the presence of increasing amounts of the HRS3-scFv-hi-IL-12 fusion protein. The cells were extensively washed and $5 \times 10^4$ cells/well were cocultivated with preactivated lymphocytes ($1 \times 10^5$ cells/well) in round bottom microtiter plates.

(B) CD30$^+$ L540 (gray bars) and CD30$^-$ BL60 cells (black bars; $1 \times 10^6$ cells each) were preincubated in the presence of the HRS3-scFv-hi-IL-12 fusion protein (100 ng/ml) or IL-12 (10 ng/ml), washed extensively and $5\times10^4$ cells/well were cocultivated with preactivated lymphocytes ($1\times10^5$/well) in round bottom microtiter plates. In a parallel assay, CD30$^+$ L540 and CD30$^-$ BL60 cells (each $5\times10^4$ cells/well), respectively, were cocultivatedwith preactivated lymphocytes ($1\times10^5$ cells/well) in the presence of soluble IL-12 (10 ng/ml). After incubation for 48 h, the culture supernatants were harvested and IFN-$\gamma$ secretion was detected by ELISA.

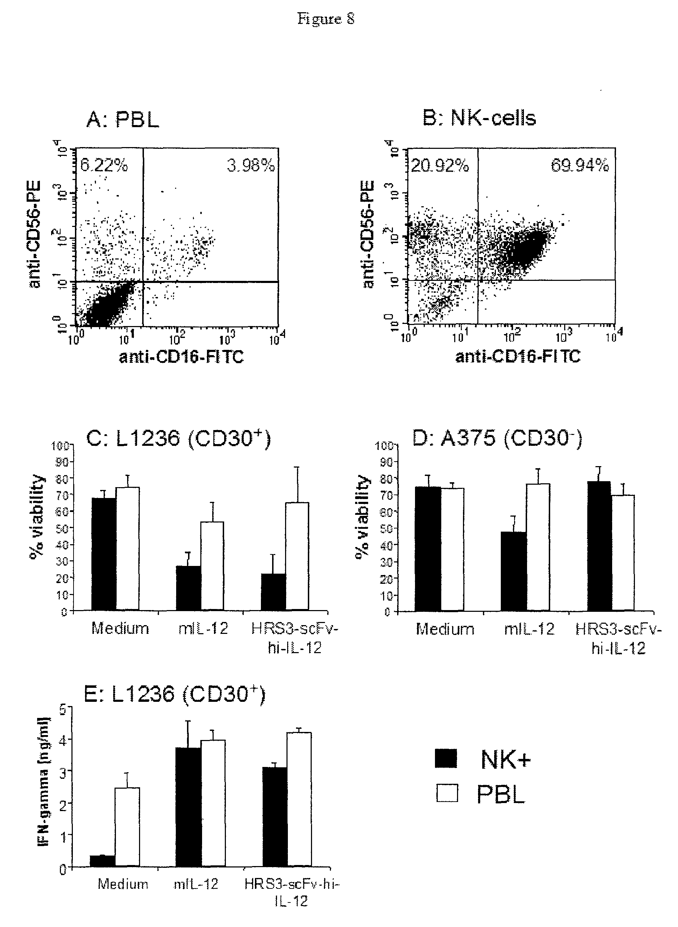

Fig. 8: HRS3-scFv-hi-IL-12 fusion protein induces NK cells to specific lysis of CD30$^+$ cells. NK cells (A) were isolated from preactivated peripheral blood lymphocytes (PBL) (B) by magnetic activated cell sorting and analysed by two colour flow cytometry utilizing a FITC-conjugated anti-CD16 and a PE-conjugated anti-CD56 mAb, respectively. CD30$^+$ L1236 cells (C) and CD30$^-$ A375 cells (D) ($1\times10^6$ each) were preincubated with culture supernatant containing HRS3-scFv-hi-IL-12 fusion protein, washed extensively and cocultivated ($5\times10^4$ cells/well) with preactivated peripheral blood lymphocytes (white bars) or highly enriched NK cells (black bars) (each $1\times10^5$ cells/well) for 48 h in round bottom microtiter plates. In a parallel assay, peripheral blood lymphocytes and NK cells, respectively, were cocultivated with CD30$^+$ L1236 (C) or CD30$^-$ A375 (D) cells in the presence of IL-12 (10 ng/ml). Viability of target cells was determined by a XTT-assay as described in Materials and Methods. (E) Supernatants of (C) were harvested after 48 h of cocultivation and IFN-$\gamma$ was detected by ELISA.

**Examples:**

To revert a Th2 polarization in HD patients the inventors generated a recombinant antibody-IL-12 cytokine fusion protein for specific targeting of IL-12 directly to the malignant H/RS cell. Since H/RS cells highly express the CD30 antigen [24, 25] that was proofed to be well suited for antibody based specific immunotherapy [26, 27], the inventors generated a fusion protein consisting of a CD30 binding domain and murine IL-12. The CD30 antigen binding domain is a single chain fragment of variable regions (scFv) derived from the anti-CD30 mAb HRS3 [28, 29] that is fused via the human IgG1 hinge region to the N-terminus of the IL-12 p40 subunit. The IL-12 p40 subunit, in turn, is linked itself to the IL-12 p35 subunit. It is demonstrated herein that the resulting antibody-cytokine fusion protein binds to both the CD30 antigen and the IL-12 receptor, and upon binding to CD30$^+$ cells activates human T cells to IFN-$\gamma$ secretion and NK cells to specific cytolysis of Hodgkin's lymphoma cells making the fusion protein attractive for the immunotherapy of Hodgin's disease.

The whole sequence of this fusion protein is shown as SEQ ID NO: 4 herein.

**MATERIALS AND METHODS**

Antibodies and reagents. The fluorescein-isothiocyanate-(FITC)-conjugated anti-CD16 mAb 3G8, the phycoerythrin-(PE)-conjugated anti-CD56 mAb B159, the PE-conjugated anti-human IL-12R$\beta$1 mAb 2.4E6, the anti-mouse IL-12 mAb C15.6, the biotinylated anti-mouse IL-12 mAb C17.8, the anti-human IFN-$\gamma$ antibody mAb NIB42, the biotinylated anti-human IFN-$\gamma$ mAb 4S.B3, and non-conjugated as well as fluorochrom-conjugated isotype control mAbs, were purchased from BD Biosciences, Heidelberg, Germany. Phycoerythrin (PE)- and peroxidase-conjugated streptavidin were purchased from Roche Diagnostics, Mannheim, Germany. The FITC-conjugated F(ab)$_2$ anti-mouse IgG was purchased from Southern Biotechnology, Birmingham, AL, USA. The hybridoma cell line HRS3 produces the anti-CD30 mAb HRS3 [29]. The CD30-specific single chain antibody HRS-scFv was derived from the anti-CD30 mAb HRS3 as described earlier [28]. The anti-HRS3 idiotypic mAb 9G10 and the recombinant CD30-human Fc fusion protein were previously described [30, 31]. The CD30-Fc fusion protein was purified from supernatants of transfected CHO cells by affinity chromatography on anti-human IgG agarose (Sigma, Deisenhofen). Monoclonal antibodies were purified from hybridoma supernatants by affinity chromatography on anti-mouse IgG agarose (Sigma). Murine IL-12 was purchased from BD Biosciences, Heidelberg, Germany.

Cell lines. L1236 and L540 are CD30$^+$ human tumor cell lines derived from Hodgkin's lymphoma, BL60 is a CD30-negative Burkitt's lymphoma-derived cell line [32-34]. The melanoma cell line A375 (ATCC CRL 1619) and the hybridoma cell line OKT3 (ATCC CRL 8001), that produces the anti-CD3 mAb OKT3, were obtained from ATCC, Rockville, MD, USA. The human renal carcinoma cell line 293T that expresses SV40 large T antigen was kindly provided by R. Bolhuis, Daniel Den Hood Cancer Center, Rotterdam, The Netherlands. The cells were cultured in RPMI 1640 or DMEM medium supplemented with 10% (v/v) fetal calf serum (FCS) (all Life Technologies, Paisley, UK).

Generation and expression of the HRS3-scFv-hi-IL-12 fusion protein. The DNA coding for murine single chain IL-12 (mscIL-12) was amplified by PCR techniques utilizing the plasmid pSFG-mIL12.p40.L.delta.p35 [21] and the following primer oligonucletides: mIL12-hinge-5'[GAAG<u>GATCCC</u>GCC**GAGCCCAAATCTCCTGACAAAACTCATA-CATGCCCACC** AATGTGGGAGCTGGAGAAAGACGTT] (SEQ ID NO: 2) and mIL 12-Xho-3'[ACTTCTCTCGAGTCAGGCGGAGCTCAGATAGCC] (SEQ ID NO: 3). Herewith, the sequence for mscIL-12 was flanked with sequences coding for the human IgG1 hinge region (bold) and for BamHI and XhoI restriction sites (underlined), respectively. The sequence of the hinge region comprises AA 226 - 241 of the human IgG1. To prevent formation of disulphide bonds between the scFv antigen binding domain and the IgG hinge region the cysteine residue at position 230 was substituted by proline. The digested PCR product was ligated into the eukaryotic expression vector DNA pRSV-HRS3scFv-γ encoding a CD30-specific recombinant immunoreceptor [35] thereby substituting the FcεRIγ signalling domain by the recombinant mscIL-12 cDNA. The resulting expression construct was termed pRSV-HRS3scFv-hi-scIL12 (Fig. 1). For transient expression of HRS3-scFv-hi-IL-12 and mscIL-12, respectively, 293T cells were transfected by calcium phosphate coprecipitation with 10-20 µg of the recombinant plasmid DNA pRSV-HRS3scFv-hi-scIL12 or pSFG-mIL12.p40.L.delta.p35, respectively. The culture supernatant was harvested 48-72 h after transfection. For stable expression of HRS3-scFv-Fc-hi-IL-12, 293T cells were co-transfected with 12 µg pRSV-HRS3scFv-hi-scIL12 DNA and 6 µg phyg DNA encoding the hygromycin resistance gene. Stably transfected cells were selected in the presence of 200 µg/ml hygromycin B and cell clones were analysed by ELISA for secretion of the fusion protein as described below.

Detection of the HRS3scFv-hi-scIL12 fusion protein. Serial dilutions of cell culture supernatants containing mscIL-12 or the HRS3-scFv-hi-IL-12 fusion protein, respectively, were incubated in microtiter plates coated with 1 µg/ml of either the anti-HRS3 idiotypic mAb 9G10, an isotype IgG1 control antibody, CD30-Fc, or an anti-mouse IL-12 mAb. Bound proteins were detected by a biotinylated anti-mouse IL-12 antibody (0.5 µg/ml) and visualized by peroxidase-coupled streptavidin (1:10,000) and ABTS® as substrate (Roche Diagnostics, Mannheim, Germany). Binding specificity of the HRS3-scFv-hi-IL-12 fusion protein was confirmed by binding competition with the parental mAb HRS3. Microtiter plates were coated with the anti-idiotypic mAb 9G10 or CD30-Fc (1 µg/ml), respectively, and HRS3-scFv-hi-IL-12 in the supernatant of transfected 293T cells (1:5) was incubated with increasing amounts of the mAb HRS3 or an IgG1 control mAb (0.01-10 µg/ml). Bound HRS3-scFv-hi-IL-12 fusion protein was detected as described above.

Purification of the HRS3scFv-hi-scIL12 fusion protein. The HRS3scFv-hi-scIL12 fusion protein was purified from cell culture supernatants by affinity chromatography utilizing the sepharose-coupled anti-HRS3 idiotypic mAb 9G10. Briefly, mAb 9G10 (1 mg/ml in PBS, pH 7.8) was incubated for 1 h at 37°C with N-hydroxy-succinimide-ester-(NHS)-activated sepharose (Amersham Biosciences, Freiburg, Germany) according to the manufacturer's recommendation. Cell culture supernatants of pRSV-HRS3-scFv-hi-IL-12 DNA transfected 293T cells were incubated with sepharose coupled 9G10 mAb and bound material was eluted with 0.1 M glycin, pH 3.0. The eluted protein was dialysed against PBS, pH 7.4, and the amount of protein was determined utilizing the Advanced Protein Assay Reagent™ (Cytoskeleton, Denver, CO, USA) according to the manufacturer's recommendation.

SDS PAGE and Western-Blot analysis. Supernatant containing HRS3-scFv-hi-IL-12 was electrophoretically separated in a 8% polyacrylamide gel under reducing and non-reducing conditions, respectively, and blotted onto nitrocellulose membrane. The membrane was probed with a biotinylated anti-mouse IL-12 mAb (0.1 µg/ml) and streptavidin-POD (1: 10,000), respectively, and bands were visualized by chemoluminescence utilizing the "ECL Western blotting detection system" (Amersham Biosciences, Freiburg, Germany). The purified HRS3-scFv-hi-IL-12 fusion protein was analysed under non-reducing conditions utilizing 4-15% polyacrylamid gradient gels in an automated electrophoresis system and visualized by the Plus One Silver Staining Kit ™ (all Amersham Biosciences).

Isolation of peripheral blood lymphocytes (PBL) and NK cells. PBL from healthy donors were isolated by density centrifugation from the peripheral blood specimen and monocytes were depleted by plastic adherence procedures. Non-adherent lymphocytes were cultured for 24 h in RPMI 1640 medium supplemented with 10% (v/v) FCS, 400 U/ml IL-2, and 100 ng/ml anti-CD3 mAb OKT3. Activated lymphocytes were washed twice and cultured for additional 48 h in RPMI 1640 medium containing 10% (v/v) FCS, 400 U/ml IL-2. CD16+CD56+ NK cells were highly enriched from activated PBL by magnetic activated cell sorting (MACS) utilizing the NK cell isolation kit (Milteny, Bergisch Gladbach, Germany) according to the manufacturer's recommendations. The number of CD16+CD56+NK cells was determinded by flow cytometry as described below. All cells were washed at least three times before experimental use.

Immunofluorescence analyses. The number of NK cells was determined by two colour flow cytometry utilizing FITC-conjugated anti-CD16 and PE-conjugated anti-CD56 mAbs. Binding of the HRS3-scFv-hi-IL-12 fusion protein to membrane-bound CD30 was monitored as follows: CD30+ L540 or CD30- BL60 cells (5x10^5) were incubated with supernatant containing HRS3-scFv-hi-IL-12 protein for 30 min on ice. Bound fusion protein was detected by biotin-conjugated anti-mouse IL-12 mAb (5 µg/ml) and PE-conjugated streptavidin (1 µg/ml). Antigen specific binding of the HRS3-scFv-hi-IL-12 protein to L540 cells was confirmed by competition with 20 µg/ml of the anti-CD30 mAb HRS3 or an isotype matched IgG1 control mAb. Binding of the HRS3-scFv-hi-IL-12 protein to the IL-12 receptor was demonstrated utilizing quiescent and OKT3 plus IL-2-activated PBL, respectively. Briefly, 5x10^5 activated or quiescent lymphocytes were incubated with supernatant containing HRS3-scFv-hi-IL-12 protein for 30 min on ice and bound fusion protein was detected by incubation with 10 µg/ml of the anti-HRS3 idiotypic mAb 9G10 or an isotype matched control IgG1 mAb, respectively, and a FITC-conjugated F(ab)'$_2$ anti-mouse antibody. Upregulation of IL-12 receptor on activated T cells was monitored by incubation with a PE-conjugated anti-mouse IL-12 mAb. Immunofluorescence was analysed using a FACScan™ cytofluorometer equipped with the CellQuest research software (Becton Dickinson, Mountain View, CA).

Activation of T cells and NK cells. OKT3 mAb (100 µg/ml) plus IL-2 (400 U/ml) preactivated T cells (1x10^5

cells/well) were cultivated for 48 h in 96-well flat bottom plates in the presence of the purified HRS3-scFv-hi-IL-12 fusion protein or mouse IL-12, respectively (BD Biosciences). In a second set of analysis, CD30$^+$ L540 and CD30$^-$ BL60 tumor cells (each 5x10$^4$ cells/well) were incubated for 60 min on ice with purified HRS3-scFv-hi-IL-12 fusion protein or IL-12, respectively. Cells were washed three times with PBS and incubated with activated PBL (1x10$^5$ cells/well) for 48 h in round bottom microtiter plates. The supernatants were harvested and analysed for IFN-γ by ELISA. Briefly, IFN-γ in the supernatant was bound to the solid phase anti-human IFN-γ mAb NIB42 (1 µg/ml) and detected by the biotinylated anti-human IFN-γ mAb 4S.B3 (0.5 µg/ml). The reaction product was visualized by a peroxidase-streptavidin-conjugate (1: 10,000) and ABTS®.

HRS3-scFv-hi-IL-12 induced NK cell cytotoxicity was monitored utilizing a XTT based colorimetric assay according to Jost et al. [36]. Briefly, CD30$^+$ L540 or CD30$^-$ A375 tumor cells (each 1x10$^6$) were incubated with supernatant containing HRS3-scFv-hi-IL-12 protein for 60 min on ice. Cells were washed 5 times with PBS and cocultivated (each 5x10$^4$ cells/well) with preactivated PBL or highly enriched NK cells. As controls, CD30$^+$ and CD30$^-$ tumor cells were cocultured with PBL and NK cells in the presence or without IL-12 (1 ng/ml). After 48 hrs, supernatants were harvested, tested for IFN-γ in the culture medium as described above. To monitor cell viability, XTT (2,3-bis(2-methoxy-4-nitro-5sulphonyl)-5[(phenyl-amino)carbonyl]-2H-tetrazolium hydroxide) reagent (1 mg/ml) (Cell Proliferation Kit II, Roche Diagnostics) was added to the cells and incubated for 30-90 min at 37°C. Reduction of XTT to formazan by viable tumor cells was monitored colorimetrically at an adsorbance wavelength of 450 nm and a reference wavelength of 650 nm. Maximal reduction of XTT was determined as the mean of 6 wells containing tumor cells only, the background as the mean of 6 wells containing RPMI 1640 medium, 10% (v/v) FCS. The non-specific formation of formazan due to the presence of effector cells was determined from triplicate wells containing effector cells in the same number as in the corresponding experimental wells. The number of viable tumor cells was calculated as follows:

$$\% \text{ viability} = \frac{OD_{(\text{exp. wells-corresponding number of effector cells})}}{OD_{(\text{tumor cells without effectors - medium})}} \times 100$$

Statistical analysis was performed utilizing Student's T test and results were regarded significant at p>0.05.

**RESULTS**

<u>The recombinant HRS3-scFv-hi-IL-12 fusion protein binds specifically to the CD30 antigen and IL,-12 receptor.</u> To combine the binding specificity for the CD30 antigen with the immune modulatory activity of IL-12 we generated an anti-CD30-scFv-IL-12 antibody-cytokine fusion protein by linking the DNA coding for the anti-CD30 single chain fragment HRS3-scFv via the human IgG hinge region to the DNA for murine single chain IL-12 (p40-p35) (Fig. 1). The DNA was expressed by the RSV LTR driven expression vector pRSV-HRS3-scFv-hi-IL-12 in 293T cells to secrete the fusion protein into the culture medium.

The culture supernatants containing the HRS3-scFv-hi-IL-12 fusion protein were analysed by ELISA for simultanous binding of the fusion protein to an anti-HRS3 idiotypic antibody and the CD30 antigen, respectively, and to an anti-mouse IL-12 antibody. The supernatant of pRSV-HRS3-scFv-hi-IL-12 DNA transfected cells binds to the immobilized CD30 antigen (Fig. 2A), the anti-HRS3 idiotypic mAb 9G10 (Fig. 2B), and to the anti-IL-12 mAb (Fig. 2C) whereas the supernatant ofpSFG-mIL12.p40.L.delta.p35 DNA transfected cells, that secrete only the IL-12 domain, binds solely to the anti-IL-12 mAb (Fig. 2C) but not to the CD30 antigen or the 9G10 mAb (Fig. 2A,B). As control, no binding was monitored on a mouse IgG1 isotype control antibody (Fig. 2D). To further confirm the binding specificity of the newly generated HRS3-scFv-hi-IL-12 fusion protein, we competed binding of the fusion protein to the CD30 antigen by the parental mAb HRS3. As demonstrated in Fig. 3, increasing amounts of mAb HRS3, but not of an isotype control mAb, inhibits binding of HRS3-scFv-hi-IL-12 fusion protein to CD30 antigen and the HRS3 idiotypic mAb 9G10.

We monitored binding of the recombinant HRS3-scFv-hi-IL-12 fusion protein to CD30$^+$ L540 tumor cells by flow cytometry utilizing an anti-IL-12 antibody. As shown in Fig. 4A, the fusion protein binds to CD30$^+$ L540 cells whereas no signal was detected on CD30$^-$ BL60 tumor cells (Fig. 4B). Binding of the fusion protein to CD30$^+$ L540 cells is specific because it was blocked in the presence of the parental anti-CD30 mAb HRS3 (Fig. 4C) but not of an isotype matched IgG1 (Fig. 4D). We furthermore monitored binding of the fusion protein to the IL-12 receptor (IL-12R) on blood lymphocytes. Because expression of the high affinity IL-12R on T cells requires cellular activation [37] (Fig. 4E,F), we compared binding of the HRS3-scFv-hi-IL-12 fusion protein to anti-CD3/IL-2 preactivated peripheral blood lymphocytes with binding to resting lymphocytes from the same donor. As shown in Fig. 4G,H the HRS3-scFv-hi-IL-12 fusion protein binds specifically on activated but not on resting lymphocytes as detected by the anti-HRS3-idiotypic mAb 9G10. Binding of the HRS3-scFv-hi-IL-12 fusion protein to activated lymphocytes is mediated through the IL-12 domain and not through its CD30 binding domain because detection of the fusion protein by the anti-idiotypic mAb 9G10, that carries an internal image of the CD30 antigen [30], requires a free HRS3 combining site for specific binding. These data demonstrate that the recombinant HRS3-scFv-hi-IL-12 fusion protein has retained specific binding to both the CD30 antigen on tumor cells and the IL-12R on activated lymphocytes.

Western-Blot analysis and purification of the HRS3-scFv-hi-IL-12 fusion protein. We analysed HRS3-scFv-hi-IL-12 containg supemantants by Western blotting under reducing and non-reducing conditions, respectively, utilizing an anti-mouse IL-12 antibody. Figure 5A demonstrates that HRS3-scFv-hi-IL-12 fusion protein is secreted as a single polypeptide chain with a molecular mass of about 110 kD corresponding to the calculated size of the monomeric protein (Fig. 5A). We purified the HRS3-scFv-hi-IL-12 fusion protein from cell culture supernatants by affinity chromatography on the anti-idiotypic mAb 9G10 immobilized on sepharose as described in Materials and Methods. The eluted protein has a mol. weight of about 110 kD under non-reducing conditions (Fig. 5B) corresponding to the results of the Western blot analysis (Fig. 5A).

The HRS3-scFv-hi-IL-12 fusion protein induces IFN-γ secretion of activated T cells. To compare the bioactivity of the fusion protein with that of IL-12, activated T cells were incubated in serial dilutions with equimolar concentrations of HRS3-scFv-hi-IL-12 or IL-12, respectively. As shown in Fig. 6, the HRS3-scFv-hi-IL-12 fusion protein induces high IFN-γ secretion in activated T cells in a dose dependent manner indicating the specific biological activity of the fusion protein. Compared to IL-12 in this assay, however, the biological activity of the fusion protein is about 5-10 times lower with respect to induction of IFN-γ secretion (Fig. 6).

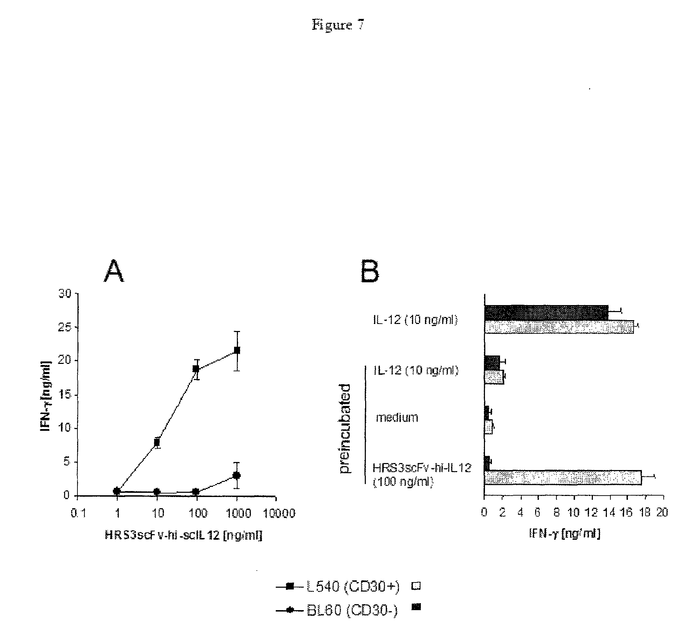

We asked whether the HRS3-scFv-hi-IL-12 fusion protein upon binding to CD30$^+$ cells can stimulate cellular activation via the IL-12R. CD30$^+$ L540 and CD30$^-$ BL60 tumor cells, respectively, were preincubated with HRS3-scFv-hi-IL-12 (1-1,000 ng/ml) or IL-12 (10 ng/ml), respectively, washed extensively and cocultured in microtiter plates (1x10$^5$ cells/well) with activated lymphocytes (1x10$^5$ cells/well) for 48h. Upon cocultivation we determined the IFN-γ content of the supernatant. As shown in Fig. 7A,B, HRS3-scFv-hi-IL-12 specifically bound on CD30$^+$ L540 cells induces IFN-γ secretion of T cells, but not upon preincubation with CD30$^-$ BL60 cells. IFN-γ induction by cell bound HRS3-scFv-hi-IL-12 fusion protein is dose dependent. As control, addition of soluble IL-12 during cocultivation of activated lymphocytes with L540 or BL60 tumor cells induced IFN-γ secretion. However, tumor cells that were only preincubated with IL-12 did not induce IFN-γ secretion (Fig. 7B).

HRS3-scFv-hi-IL-12 induces NK cell, but not T cell mediated cytolysis of CD30$^+$ tumor cells. We asked whether the HRS3-scFv-hi-IL-12 fusion protein after binding to CD30$^+$ cells induces NK and T cells to specific lysis of the CD30$^+$ tumor cell. We therefore isolated NK cells from peripheral blood lymphocytes (PBL) that were OKT3/IL-2 preactivated by negative MACS fractionation providing a highly enriched CD 16$^+$CD56$^+$ NK cell population (Fig. 8A,B). Unfractionated PBMC and isolated NK cells, respectively, were cocultivated with CD30$^+$ L1236 and CD30$^-$ A375 tumor cells, respectively, in an effector: target cell (E:T) ratio of 2:1. Tumor cells were preincubated with the HRS3-scFv-hi-IL-12 fusion protein and subsequently washed intensively prior to administration to the assay. Alternatively, the assay was run in the presence of soluble IL-12. After coincubation with PBL or NK cells, the viability of tumor cells was determined by a XTT-based colorimetric assay as described in Materials and Methods. As demonstrated in Fig. 8 C, D, CD30$^+$ L1236 cells coated with HRS3-scFv-hi-IL-12 induced NK cells to highly efficient lysis of the CD30$^+$ target cells whereas CD30$^-$ A375 tumor cells that do not bind the fusion protein did not. As control, both A375 cells as well as L1236 cells were lysed by NK cells upon addition of soluble IL12. Unfractionated PBL, that predominantly harbor T cells, did not significantly lyse CD30$^+$ L1236 or CD30$^-$ A375 cells in the presence of soluble IL-12 or of cell bound HRS3-scFv-hi-IL-12. However, soluble IL-12 and CD30-immobilized HRS3-scFv-hi-IL-12 fusion protein induced IFN-γ secretion in both NK and PBL cells (Fig. 8E). These data indicate that the HRS3-scFv-hi-IL-12 fusion protein induces NK cells, but not T cells in the PBL fraction, to specific cytolysis of CD30$^+$ tumor cells.

Antibody-targeted delivery of Th1 immunostimulatory cytokines like IL-2 and, moreover, IL-12 to tumor tissues opens novel strategies for the site specific generation of an anti-tumor immune response. To deliver IL-12 to CD30$^+$ lymphoma cells, we generated an anti-CD30-IL-12 antibody-cytokine fusion protein that consists of an anti-CD30 scFv antibody for specific targeting and of mouse IL-12 for locally modulating the immune response. To ensure functional activity of the domains in a recombinant fusion protein, the anti-CD30 antibody derived VH and VL regions were joined together by a flexible (Gly$_4$Ser)$_3$ linker to form the anti-CD30 scFv, as were the p40 and p35 subunits to form the single chain IL-12. Both domains, scFv and single chain IL-12, were linked together via the human IgG1 hinge region resulting in the recombinant HRS3-scFv-hi-IL-12 protein that is expressed as a single, monomeric polypeptide chain. It was demonstrated that the HRS3-scFv-hi-IL-12 fusion protein exhibits both highly specific binding to the CD30 antigen and the immunostimulatory potency of IL-12.

The ADDINADDINADDINHRS3-scFv-hi-IL-12 fusion protein presented herein may be suitable as an anti-tumor reagent for the immunotherapy of Hodgkin's lymphoma. Since Hodgkin's lymphoma has been described as an acquired cellular immune deficiency involving several Th2 cytokines [17], tissue specific delivery of IL-12 via the HRS3-scFv-hi-IL-12 fusion protein may counterbalance the disturbed T cell immunity. Accordingly, we here demonstrate that the HRS3-scFv-hi-IL-12 fusion protein when bound to the cell surface of CD30$^+$ Hodgkin's lymphoma cells can induce peripheral blood T cells to high IFN-γ secretion. T cell activation via the IL-12R, however, does not result in enhanced tumor cell lysis of Hodgkin's lymphoma derived tumor cells in vitro (cf. Fig. 8). On the other hand, the presence of high amounts of IFN-γ was demonstrated to be crucial for an efficient anti-tumor response in vivo [38, 39] indicating a more complex regulation of IFN-γ mediated anti-tumor reactivity. NK cells complement T cell immunity and lyse tumor targets in a MHC-molecule independent fashion that is highly enhanced by IL-12 [40]. We accordingly found that, after binding to CD30⁺ cells the HRS3-scFv-hi-IL-12 fusion protein drives NK cells, in contrast to T cells, to an efficient cytolysis of CD30⁺ lymphoma cells. These findings have several implications for the immunotherapy of Hodgkin's disease with the HRS3-scFv-hi-IL-12 fusion protein: (i) Upon binding to Hodgkin's lymphoma cells, the HRS3-scFv-hi-IL-12 fusion protein induces high secretion of IFN-γ in both T cells and NK cells that combined with the IL-12 reactivity of the fusion protein may shift the Th2 inbalance in the Hodgkin lesion towards a Th1 reactivity. (ii) The HRS3-scFv-hi-IL-12 protein mediated recruitment of NK cells for MHC-independent tumor cell lysis may counterbalance a potential T cell anergy and may open new pathways for the specific immunotherapy of Hodgkin's lymphoma.

**REFERENCES**

1. Trinchieri, G. and P. Scott, *Interleukin-12: a proinflammatory cytokine with immunoregulatory functions.* Res Immunol, 1995. **146**(7-8): p. 423-31.

2. Thomas, M.J., et al., *CD8 T cells inhibit IgE via dendritic cell IL-12 induction that promotes Th1 T cell counter-regulation.* J Immunol, 2002. **168**(1): p. 216-23.

3. D'Orazio, T.J. and J.Y. Niederkorn, *A novel role for TGF-beta and IL-10 in the induction of immune privilege.* J Immunol, 1998. **160**(5): p. 2089-98.

4. Corinti, S., et al., *Regulatory activity of autocrine IL-1 0 on dendritic cell functions.* J Immunol, 2001. **166**(7): p. 4312-8.

5. Poppema, S. and A. van den Berg, *Interaction between host T cells and Reed-Sternberg cells in Hodgkin lymphomas.* Semin Cancer Biol, 2000. **10**(5): p. 345-50.

6. Nagai, H., et al., *Elimination of CD4(+) T cells enhances anti-tumor effect of locally secreted interleukin-12 on B16 mouse melanoma and induces vitiligo-like coat color alteration.* J Invest Dermatol, 2000. **115**(6): p. 1059-64.

7. Seo, N. and Y. Tokura, *Downregulation of innate and acquired antitumor immunity by bystander gammadelta and alphabeta T lymphocytes with Th2 or Tr1 cytokine profiles.* J Interferon Cytokine Res, 1999. **19**(6): p. 555-61.

8. Seo, N., et al., *Depletion of IL-10- and TGF-beta-producing regulatory gamma delta T cells by administering a daunomycin-conjugated specific monoclonal antibody in early tumor lesions augments the activity of CTLs and NK cells.* J Immunol, 1999. **163**(1): p. 242-9.

9. Kadin, M.E., *Pathology of Hodgkin's disease.* Curr Opin Oncol, 1994. **6**(5): p. 456-63.

10. Kuppers, R. and K. Rajewsky, *The origin of Hodgkin and Reed/Sternberg cells in Hodgkin's disease.* Annu Rev Immunol, 1998. **16**: p. 471-93.

11. Skinnider, B.F. and T.W. Mak, *The role ofcytokines in classical Hodgkin lymphoma.* Blood, 2002. **99**(12): p. 4283-97.

12. Peh, S.C., L.H. Kim, and S. Poppema, *TARC, a CC chemokine, is frequently expressed in classic Hodgkin's lymphoma but not in NLP Hodgkin's lymphoma, T-cell-rich B-cell lymphoma, and most cases of anaplastic large cell lymphoma.* Am J Surg Pathol, 2001. **25**(7): p. 925-9.

13. Poppema, S., et al., *Immune escape mechanisms in Hodgkin's disease.* Ann Oncol, 1998. **9 Suppl 5**: p. S21-4.

14. Newcom, S.R., A.A. Ansari, and L. Gu, *Interleukin-4 is an autocrine growth factor secreted by the L-428 Reed-Sternberg cell.* Blood, 1992. **79**(1): p. 191-7.

15. Skinnider, B.F., et al., *Interleukin 13 and interleukin 13 receptor are frequently expressed by Hodgkin and Reed-Sternberg cells of Hodgkin lymphoma.* Blood, 2001. **97**(1): p. 250-5.

16. Chan, W.C., *The Reed-Sternberg cell in classical Hodgkin's disease.* Hematol Oncol, 2001. **19**(1): p. 1-17.

17. van den Berg, A., L. Visser, and S. Poppema, *High expression of the CC chemokine TARC in Reed-Sternberg cells. A possible explanation for the characteristic T-cell infiltratein Hodgkin's lymphoma.* Am J Pathol, 1999. **154**(6): p. 1685-91.

18. Bohlen, H., et al., *Poor clinical outcome ofpatients with Hodgkin's disease and elevated interleukin-10 serum levels. Clinical significance of interleukin-10 serum levels for Hodgkin's disease.* Ann Hematol, 2000. **79**(3): p. 110-3.

19. Gillies, S.D., et al., *Antibody-IL-12 fusion proteins are effective in SCID mouse models of prostate and colon carcinoma metastases.* J Immunol, 1998. **160**(12): p. 6195-203.

20. Peng, L.S., M.L. Penichet, and S.L. Morrison, *A single-chain IL-12 IgG3 antibody fusion protein retains antibody specificity and IL-12 bioactivity and demonstrates antitumor activity.* J Immunol, 1999. **163**(1): p. 250-8.

21. Lieschke, G.J., et al., *Bioactive murine and human interleukin-12 fusion proteins which retain antitumor activity in vivo.* Nat Biotechnol, 1997. **15**(1): p. 35-40.

22. Gillies, S.D., et al., *Bi-functional cytokine fusion proteins for gene therapy and antibody-targeted treatment of cancer.* Cancer Immunol Immunother, 2002. **51**(8): p. 449-60.

23. Halin, C., et al., *Enhancement of the antitumor activity of interleukin-12 by targeted delivery to neovasculature.* Nat Biotechnol, 2002. **20**(3): p. 264-9.

24. Anagnostopoulos, I., et al., *European Task Force on Lymphoma project on lymphocyte predominance Hodgkin disease: histologic and immunohistologic analysis of submitted cases reveals 2 types of Hodgkin disease with a nodular growth pattern and abundant lymphocytes.* Blood, 2000. **96**(5): p. 1889-99.

25. von Wasielewski, R., et al., *Lymphocyte-predominant Hodgkin's disease. An immunohistochemical analysis of 208 reviewed Hodgkin's disease cases from the German Hodgkin Study Group.* Am J Pathol, 1997. **150**(3): p. 793-803.

26. Hombach, A., et al., *T cells engrafted with a recombinant anti-CD30 receptor target autologous CD30(+) cutaneous lymphoma cells.* Gene Ther, 2001. **8**(11): p. 891-5.

27. Hartmann, F., et al., *Treatment of refractory Hodgkin's disease with an anti-CD16/CD30 bispecific antibody.* Blood, 1997. **89**(6): p. 2042-7.

28. Hombach, A., et al., *Isolation of single chain antibody fragments with specificity for cell surface antigens by phage display utilizing internal image anti-idiotypic antibodies.* J Immunol Methods, 1998. **218**(1-2): p. 53-61.

29. Engert, A., et al., *Antitumor effects of ricin A chain immunotoxins prepared from intact antibodies and Fab'fragments on solid human Hodgkin's disease tumors in mice.* Cancer Res, 1990. **50**(10): p. 2929-35.

30. Pohl, C., et al., *CD30-specific AB1-AB2-AB3 internal image antibody network: potential use as anti-idiotype vaccine against Hodgkin's lymphoma.* Int J Cancer, 1993. **54**(3): p. 418-25.

31. Renner, C., et al., *The role of lymphocyte subsets and adhesion molecules in T cell-dependent cytotoxicity mediated by CD3 and CD28 bispecific monoclonal antibodies.* Eur J Immunol, 1995. **25**(7): p. 2027-33.

32. Wolf, J., et al., *Peripheral blood mononuclear cells of a patient with advanced Hodgkin's lymphoma give rise to permanently growing Hodgkin-Reed Sternberg cells.* Blood, 1996. **87**(8): p. 3418-28.

33. Lenoir, G., M., Philip, T., Sohier, R., *Burkitt type lymphoma-EBV association and cytogenetic markers in cases from various geographic locations.,* in *Pathogenesis of leukemias and lymphomas: Environmental Influences,* I. Magrath, T., O'Conor, G., T., Ramot, B., Editor. 1994, Raven Press: New York. p. 283.

34. Diehl, V., et al., *Hodgkin's disease cell lines: characteristics and biological activities.* Hamatol Bluttransfus, 1983. **28**: p. 411-7.

35. Hombach, A., et al., *An anti-CD30 chimeric receptor that mediates CD3-zeta-independent T-cell activation against Hodgkin's lymphoma cells in the presence of soluble CD30.* Cancer Res, 1998. **58**(6): p. 1116-9.

36. Jost, L.M., J.M. Kirkwood, and T.L. Whiteside, *Improved short- and long-term XTT-based colorimetric cellular cytotoxicity assay for melanoma and other tumor cells.* J Immunol Methods, 1992. **147**(2): p. 153-65.

37. Desai, B.B., et al., *IL-12 receptor. II. Distribution and regulation of receptor expression.* J Immunol, 1992. **148**(10): p. 3125-32.

38. Wang, B., et al., *Genetic disruption of host interferon-gamma drastically enhances the metastasis of pancreatic adenocarcinoma through impaired expression of inducible nitric oxide synthase.* Oncogene, 2001. **20**(47): p. 6930-7.

39. Kemp, R.A. and F. Ronchese, *Tumor-specific Tc1, but not Tc2, cells deliver protective antitumor immunity.* J Immunol, 2001. **167**(11): p. 6497-502.

40. Hirschowitz, E.A., et al., *Regional treatment of hepatic micrometastasis by adenovirus vector-mediated delivery of interleukin-2 and interleukin-12 cDNAs to the hepatic parenchyma.* Cancer Gene Ther, 1999. **6**(6): p. 491-8.

## Claims

1. A recombinant fusion protein, which comprises the following functionally linked components:

    a) a coupling domain for specific ligand binding,
    b) a single chain IL-12 or a fragment thereof, wherein the fragment at least contains the p40 subunit of IL-12, and
    (c) optionally a peptide linker, which links components (a) and (b) to each other,

    wherein the single chain IL-12 or the fragment thereof is linked to the other components via the N-terminus of the p40 subunit.

2. The fusion protein of claim 1, wherein the coupling domain is selected from a group consisting of polypeptides, antibodies, antibody fragments, therapeutic agents, dyes, toxins and chelating agents.

3. The fusion protein of claim 2, wherein the antibody fragment is a scFv, preferably an anti-CD30 scFv, more preferably the HRS3-scFv.

4. The fusion protein of one or more of claims 1-3, wherein the peptide linker is the human IgG1 hinge region.

5. An isolated nucleic acid, which is coding for the fusion protein of one or more of claims 1-4.

6. A nucleic acid, which is coding for a recombinant fusion protein and having the nucleic acid sequence of SEQ ID NO: 4.

7. An isolated nucleic acid, which is a transcriptional product of the nucleic acid of claim 5 or 6, or which selectively hybridizes to the nucleic acids of claims 5 or 6 under moderately stringent conditions.

8. A fusion protein, which is encoded by a nucleic acid of one of claims 5-7.

9. A vector, which comprises one or more of the nucleic acids of claims 5-7.

10. An expression vector, which comprises one or more of the nucleic acids of claims 5-7 and one or more regulatory sequences.

11. The vector of claim 9 or 10, which is a plasmid, cosmid, bacteriophage or a virus.

12. A host cell, which has been transformed with the vector of any of claims 9-11.

13. The host cell of claim 12, which is a eukaryotic cell, preferably a mammalian cell, plant cell, yeast cell or an insect cell, or a prokaryotic cell, preferably *E. coli* or *Bacillus subtilis.*

14. The mammalian cell of claim 13, which is a CHO, COS, HeLa, 293T, HEH or BHK cell.

15. A pharmaceutical composition comprising a therapeutically effective amount of the fusion protein of one or more of claims 1-4 or 8, of the nucleic acids of one or more of claims 5-7 or of the vectors of one or more of claims 9-11, and a pharmaceutically acceptable carrier.

16. The pharmaceutical composition of claim 15, wherein the fusion protein is further coupled to a therapeutic agent, preferably to a radioisotope, a biological response modifier, e. g. IL-2, IL-4, TNF-alpha, enzymes, e. g. for prodrug activation, coupling domains for engraftment of radioisotypes and drugs, e. g. cytostatic drugs.

17. The pharmaceutical composition of claim 15 or 16, which is a vaccine.

18. Use of the pharmaceutical compositions of one or more of claims 15 to 17 for the manufacture of a medicament for the immunotherapy of malignant diseases, preferably the Hodgkin lymphoma, minimal residual disease, inflammatory diseases, allergic and rheumatoid diseases, tumor prevention, the immunotherapy of preneoplastic lesions and suppression of a Th2-immune response mediated by CD30+ immunological competent cells.

19. A diagnostic composition comprising the fusion protein of one or more of claims 1-4 or 8, wherein the fusion protein is detectably labelled, preferably radioactively labelled.

20. Use of the diagnostic composition of claim 19 in the diagnosis of malignant diseases, preferably the Hodgkin lymphoma, minimal residual disease, inflammatory diseases, allergic and rheumatoid diseases, preneoplastic lesions and of a Th2-immune response mediated by CD30+ immunological competent cells.

21. A method of producing a substantially pure fusion protein of one of claims 1-4 or 8, comprising transforming a host cell of one of claims 12-14 with a vector of one of claims 9-11, cultivating said host cell under conditions, which allow an expression of the vector by the host cell, and isolating the fusion protein from the host cell.

22. A method of producing a substantially pure fusion protein, comprising the steps of:

   a) providing a coupling domain and a single chain IL-12 or a fragment thereof, wherein the fragment at least contains the p40 subunit of IL-12,
   b) coupling the coupling domain via a peptide bond to the N-terminus of the p40 subunit of the single chain IL-12 or the fragment thereof, and
   (c) isolating the obtained fusion protein.

Figure 1

HRS3-scFv                                                        single-chain IL-12

| VH | L | VL | EPKSPDKTHTCPP | p40 | L | p35 |

human IgG hinge

Figure 2

A: CD30

B: 9G10

C: anti-IL-12

D: IgG1

HRS3-scFv-hi-IL12    scIL-12

Figure 3

A: CD30-Fc

B: 9G10

HRS3 mAb    mIgG1 mAb

Figure 4

A: L540 (CD30⁺)
+HRS3-scFv-hi-IL-12

B: BL60 (CD30⁻)
+HRS3-scFv-hi-IL-12

C: L540 (CD30⁺)
+HRS3
+HRS3-scFv-hi-IL-12

D: L540 (CD30⁺)
+mIgG1
+HRS3-scFv-hi-IL-12

E: Resting PBL
+anti-IL-12R

F: Activated PBL
+anti-IL-12R

G: Resting PBL
+HRS3-scFv-hi-IL-12

H: Activated PBL
+HRS3-scFv-hi-IL-12

Figure 5

A

B

— 220 kD

— 220 kD

— 90 kD

— 120 kD

— 90 kD

— 50 kD

— 50 kD

1    2

1    2

Figure 6

Figure 7

**A**

IFN-γ [ng/ml] vs HRS3scFv-hi-scIL 12 [ng/ml]

**B**

preincubated

- IL-12 (10 ng/ml)
- IL-12 (10 ng/ml)
- medium
- HRS3scFv-hi-IL12 (100 ng/ml)

IFN-γ [ng/ml]

— L540 (CD30+) □
— BL60 (CD30-) ■

Figure 8

A: PBL

B: NK-cells

C: L1236 (CD30$^+$)

D: A375 (CD30$^-$)

E: L1236 (CD30$^+$)

■ NK+
□ PBL

**European Patent** | **PARTIAL EUROPEAN SEARCH REPORT** | **Application Number**
**Office** | which under Rule 45 of the European Patent Convention shall be considered, for the purposes of subsequent proceedings, as the European search report | EP 02 02 5124

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99 29732 A (LEXIGEN PHARM CORP) 17 June 1999 (1999-06-17)<br><br>* page 10, line 9 - page 12, line 15 *<br>* example 3 *<br>* figures 2,4 * | 1,2, 5-14,21, 22 | C07K14/54 C07K16/28 C07K19/00 C12N5/00 A61K38/17 A61P35/00 |
| Y | * example 7 * | 1-18,21, 22 | |
| X | KIM T S ET AL: "AN OVALBUMIN-IL-12 FUSION PROTEIN IS MORE EFFECTIVE THAN AVALBUMIN PLUS FREE RECOMBINANT IL-12 IN INDUCING A T HELPER CELL TYPE 1-DOMINATED IMMUNE RESPONSE AND INHIBITING ANTIGEN-SPECIFIC IGE PRODUCTION" JOURNAL OF IMMUNOLOGY, THE WILLIAMS AND WILKINS CO. BALTIMORE, US, vol. 158, no. 9, 1 May 1997 (1997-05-01), pages 4137-4144, XP000938818 ISSN: 0022-1767 * page 4138, left-hand column, last paragraph - right-hand column, paragraph 1; figures 1-3,5,6 * | 1,2,15, 16 | |
| | -/-- | | |

**TECHNICAL FIELDS SEARCHED (Int.Cl.7)**

C12N
C07K
A61K
A61P

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claim 20 might comprise a diagnostic method practised on the human/animal body (Article 52(4) EPC), the search has been carried out and based on the alleged effects of the compound/composition.

| Place of search | Date of completion of the search | Examiner |
|---|---|---|
| BERLIN | 8 April 2003 | ALCONADA RODRIG.., A |

EPO FORM 1503 03.82 (P04C07)

| | European Patent Office | **PARTIAL EUROPEAN SEARCH REPORT** | Application Number EP 02 02 5124 |
|---|---|---|---|

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| Y | US 5 994 104 A (ANDERSON ROBERT JAMES ET AL) 30 November 1999 (1999-11-30) * column 10, line 54-60 * --- | 1-18,21, 22 | |
| A | HOMBACH A ET AL: "Isolation of single chain antibody fragments with specificity for cell surface antigens by phage display utilizing internal image anti-idiotypic antibodies" JOURNAL OF IMMUNOLOGICAL METHODS, ELSEVIER SCIENCE PUBLISHERS B.V.,AMSTERDAM, NL, vol. 218, no. 1-2, 1 September 1998 (1998-09-01), pages 53-61, XP004146531 ISSN: 0022-1759 * page 58, right-hand column - page 59, left-hand column * ----- | 3,19,20 | TECHNICAL FIELDS SEARCHED (Int.Cl.7) |

EPO FORM 1503 03.82 (P04C10)

28

**EP 1 418 184 A1**

## ANNEX TO THE EUROPEAN SEARCH REPORT
## ON EUROPEAN PATENT APPLICATION NO.

EP 02 02 5124

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2003

| Patent document cited in search report | Publication date | Patent family member(s) | Publication date |
|---|---|---|---|
| WO 9929732 A | 17-06-1999 | AU 1716099 A<br>CA 2312188 A1<br>EP 1037927 A2<br>JP 2001525423 T<br>US 2002193570 A1<br>WO 9929732 A2 | 28-06-1999<br>17-06-1999<br>27-09-2000<br>11-12-2001<br>19-12-2002<br>17-06-1999 |
| US 5994104 A | 30-11-1999 | NONE | |